Europäisches Patentamt

(19) European Patent Office

Office européen des brevets

(11) Publication number: **0 305 506 B1**

(12) # EUROPEAN PATENT SPECIFICATION

(45) Date of publication of patent specification:
16.10.91 Bulletin 91/42

(51) Int. Cl.⁵: **B65D 83/04**

(21) Application number: **88903578.8**

(22) Date of filing: **07.03.88**

(86) International application number:
**PCT/US88/00868**

(87) International publication number:
**WO 88/06558 07.09.88 Gazette 88/20**

(54) **PACKAGE FOR OF ADMINISTERING A PHARMACEUTICAL PREPARATION.**

(30) Priority: **06.03.87 US 23093**

(43) Date of publication of application:
**08.03.89 Bulletin 89/10**

(45) Publication of the grant of the patent:
**16.10.91 Bulletin 91/42**

(84) Designated Contracting States:
**AT BE CH DE FR GB IT LI LU NL SE**

(56) References cited:
**FR-A- 1 327 208**
**FR-A- 2 198 461**
**GB-A- 2 034 662**
**US-A- 2 134 489**
**US-A- 2 968 391**
**US-A- 3 503 493**
**US-A- 3 741 384**

(56) References cited:
**US-A- 4 209 096**
**US-A- 4 212 918**
**US-A- 4 294 361**
**US-A- 4 357 192**
**US-A- 4 478 658**

(73) Proprietor: **BAKER CUMMINS
PHARMACEUTICALS, INC. (a Florida
corporation)
8800 N.W. 36th Street
Miami Florida 33178 (US)**

(72) Inventor: **HSIAO, Charles
4890 S.W. 104th Avenue
Copper City, FL 33328 (US)**

(74) Representative: **Lally, William et al
FORRESTER & BOEHMERT
Widenmayerstrasse 4/I
W-8000 München 22 (DE)**

## Description

This invention generally relates to an arrangement for, and a method of, administering a pharmaceutical preparation and, more particularly, to the administration of an orally active pharmaceutical preparation contained in non-tacky coated pellets by breaking open a frangible packet containing the pellets, and by pouring the pellets from the opened packet into a human or animal patient's mouth.

Pharmaceutical preparations are traditionally orally administered to patients in many forms. Tablets are often used, but are not altogether desirable in certain cases. For example, depending on their size and coating, tablets can be difficult for certain patients, particularly children or small animals, to swallow. In some instances, the tablets begin to dissolve immediately upon coming into contact with saliva in the mouth, causing the unpleasant taste of the medicinal preparation to be sensed. In addition, relatively small tablets can be easily concealed or stolen. This is a particular importance in the administration of methadone and like drugs to narcotic addicts, or other medications to mental patients, because such patients may sometimes not swallow the tablets, but, instead, conceal them in their mouths or clothing in order to sell or dispose of the medication.

Capsules are also commonly used, particularly in those cases where slow or sustained release of the pharmaceutical preparation is desired. Although generally satisfactory for their intended purpose, capsules which enclose the pharmaceutical preparation within a gelatin container are generally larger in size than tablets and, hence, aggravate the swallowing problem. To overcome this problem, some patients break open the capsules to swallow the pharmaceutical preparation within the gelatin container, but often the pharmaceutical preparation has a bitter taste. In any event, the breaking open of a gelatin container can be messy and cause loss of part of the dosage amount.

Liquid pharmaceutical formulations are also generally satisfactory, but often need to be refrigerated or shaken prior to use. In some instances, such as certain pediatric antibiotic suspensions, preparation of the liquid formulations require addition of water to a powder immediately prior to use. Unless measuring spoons or the like are used, the dosing of the liquid formulation may be inaccurate, and spillage is a frequent problem, particularly with children. Also, liquid formulations often contain sweeteners, coloring and flavoring agents and other additives, many of which are not acceptable to nutrition-minded patients. Liquid formulations are also not stable for lengthy periods.

Another common problem with pharmaceutical preparations in tablet or capsule form is that there is very little room, if any, to print indicia on the tablet or capsule itself. Such indicia could be very useful if they identified the preparation itself, the dosage amount, the expiration date, or provided warning notices or directions for use. To meet this need, some drug manufacturers will design a tablet with a characteristic shape or color, or imprint an identifying mark on the tablet. However, no room exists on the tablet itself for more printed information and, generally, this information would be provided on a bottle or other relatively large-sized container housing the individual tablets or capsules.

However, such large-sized bottles or containers are generally too large to fit in one's pocket and, rather than being carried about, are generally stored in one's medicine cabinet and thus are out of sight of the patient when the tablet/capsule is being orally taken. In the case where a patient takes multiple medications, the medications are often co-mingled in a pill box or similar unmarked container, whereby the medications can be identified, if at all, only by their size, shape and colour and reference to a pharmaceutical text. Elderly patients, especially, may become confused when unmarked medications are present in an unmarked holder, and may possibly take the wrong medication at the wrong time or exceed their recommend dosage of a given medication.

US-A-4209096 and FR-A-2198461 both disclose packages for administering a pharmaceutical preparation, and comprise a breakable packet having a backing sheet and a covering member overlying the backing sheet and bounding therewith a compartment in which the pharmaceutical preparation is contained, the covering member including a hollow main portion extending along a longitudinal direction, a neck portion adjacent the main body portion, a head portion adjacent to the neck portion, and a frangible zone which, when broken, opens the compartment for discharge of the pharmaceutical preparation.

However on breakage of the head, the frangible zone may produce jagged edges which restricts the usage of the package.

It is a general object of this invention to provide a novel arrangement for administering pharmaceutical preparations which avoid the aforementioned drawbacks inherent in tablet, capsule or liquid formulation.

It is another object of this invention to provide an easily-openable pack containing the pharmaceutical preparation for prompt dispensing of the preparation.

A further object of this invention is to provide a miniaturised, frangible packet small enough to be easily carried in one's pocket and large enough to bear indicia identifying, e.g. the pharmaceutical preparation, the dosage, the expiration date, warning notices, and use instructions.

Yet another object of this invention is to provide pharmaceutical preparations in the form of coated pellets which are easy to swallow or to combine with liquids or foods for oral ingestion.

Still another object of this invention is to provide the coated pellets with non-tacky coatings to prevent the pellets from adhering to one another or to their container during manufacturing, storage and/or use.

A still further object of this invention is to orally administer the pellets while concealing the taste of the pharmaceutical preparation contained therein.

Another object of this invention is to substantially prevent the pellets from dissolving immediately upon entry into a patient's mouth, yet provide for easy dissolution in the stomach for quick absorption into the bloodstream.

Yet another object of this invention is to administer the pharmaceutical preparation to persons of all ages, e.g., pediatric or geriatric patients, and to animals.

An additional object of this invention is to provide a novel form for pharmaceutical preparations administered to drug addicts and psychiatric patients which will substantially prevent theft or concealment of the medications.

Still another object of this invention is to provide a normally-sealed packet which, once its seal is broken, cannot be re-sealed, thereby preventing tampering with the medication.

Another object of this invention is to provide a readily disposable pellet-containing packet.

A further object of this invention is to provide a novel method of administration of a pharmaceutical preparation which is easy to swallow, inexpensive to manufacture, and convenient to use.

In keeping with these objects, and others which will become apparent hereinafter, one feature of this invention resides, briefly stated, in an arrangement for, and a method of, administering one or more dosage units of a pharmaceutical preparation which comprises a multitude of pellets containing the pharmaceutical preparation contained in a breakable packet. The breakable packet includes a backing sheet, preferably constituted of a paper material, and a covering member, preferably constituted of a synthetic plastic material and overlying the backing sheet. The covering member and backing sheet together bound a compartent in which the pellets are contained. The pellets have non-tacky coatings to prevent them from adhering to one another and to the packet. The packet has a frangible zone which, when broken, enables removal of the pellets from the compartment.

In use, the backing sheet is bendable when subjected to external forces, e.g., moderate fingertip pressures, at the frangible zone. When the backing sheet is bent, the covering member fractures at the frangible zone into fractured parts whereby an opening in the compartment is created, permitting removal of the pellets. The fractured parts remain on the bent backing sheet for ease of disposal.

Preferably, the covering member includes a hollow main portion having a predetermined cross-section, a neck portion having a cross-section less than said predetermined cross-section, and a flange portion sealed and attached to one major surface of the backing sheet. In the preferred embodiment, the frangible zone extends across the neck portion and, when the covering member fractures at the neck portion, walls are formed at the neck portion which bound a tapered pouring spout or funnel through which the pellets pass.

An opposite major surface of the backing sheet is advantageously applied with indicia to identify the pharmaceutical preparation itself, the dosage amount, the expiration date, and can provide warning notices or use instructions, as desired.

The covering member is preferably constituted of a light-transmissive material to permit viewing of the pellets through the covering member. In this way, a user can verify the extent to which the pellets have been removed from the packet.

Another feature of this invention resides in providing a non-tacky coating for each medication-containing pellet, said coating comprising a polymeric material, e.g., a cationic copolymeric acrylate resin based on methacrylate and neutral methacrylic acid esters. The coating further includes a basic compound filler that is soluble in acid. Any organic or inorganic compound having a high solubility rate in an acidic medium such as gastric juices may serve as the basic compound filler. As preferred embodiments, the filler may be calcium carbonate, aluminum hydroxide or magnesium carbonate, or any mixture thereof.

In use, the pellet coating surrounding the pharmaceutical preparation prevents the latter from being dissolved upon contact with saliva in a patient's mouth and, instead, delays such dissolution until the pellets reach the gastric juices in the patient's stomach. The basic compound dissolves in the acidic gastric juices and liberates the pharmaceutical preparation. Thus, the basic compound filler may be used in an amount sufficient to facilitate the desired release characteristics of the preparation. Typically, the filler is present in the coating in an amount from about 10% to about 90% by weight.

The polymeric material is advantageously selected from the group consisting of cellulosic, polyacrylate and polyvinyl alcoholic polymers.

A single packet may contain one or more compartments, each containing a fraction of a recommended dose to be administered to a patient. Likewise, multiple packets representing multiple doses can be provided on a common backing sheet or on a roll, and can be detached from the roll as required in order to allow precise dosages to be administered to the patient.

The average size of each pellet is generally not greater than 1 mm in diameter.

The novel features which are considered as characteristic of the invention are set forth in particular in the appended claims. The invention itself, however, both as to its construction and its method of operation, together with additional objects and advantages thereof, best will be understood from the following description of specific embodiments when read in connection with the accompanying drawings.

FIG. 1 is a front perspective view of an arrangement for administering a pharmaceutical preparation in accordance with this invention;

FIG. 2 is an enlarged cross-sectional view taken on line 2–2 of FIG. 1;

FIG. 3 is a greatly enlarged sectional view of one of the multitude of pellets containing the pharmaceutical preparation;

FIG. 4 is a rear view of the arrangement of FIG. 1, bearing printed indicia; and

FIG. 5 is a top plan view of another arrangement for administering a pharmaceutical preparation in accordance with this invention.

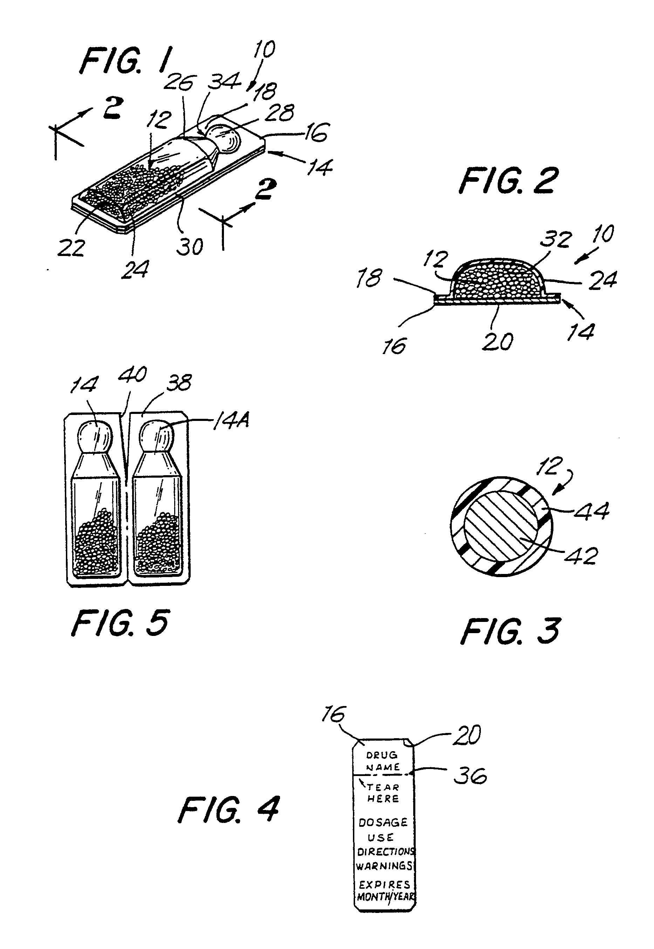

Referring now to FIGs. 1 and 2, reference numeral 10 generally identifies an arrangement for administering a pharmaceutical preparation. The arrangement 10 comprises a multitude of pellets, a representative one 12 of which is shown in enlarged sectional view in FIG. 3. The pellets 12 contain the pharmaceutical preparation to be administered to a patient.

The arrangement 10 further comprises a breakable packet 14 for holding the pellets 12. The packet includes a backing sheet 16, preferably constituted of a paper material, although vinyl and other thin, flexible sheeting materials may be utilized. The backing sheet generally, although not necessarily, lies in a plane and has opposite major surfaces, namely, top surface 18 and bottom surface 20. The paper material of the backing sheet renders the same bendable, and provides a certain degree of frangibility.

The packet 14 further includes a covering member 22, preferably constituted of a synthetic plastic material which is light-transmissive. The covering member 22 includes a hollow main portion 24 having a predetermined half-cylindrical cross-section, a neck portion 26 having a half-frusto-conical cross-section less than said cross-section of main portion 24, a head portion 28 having a half-spherical cross-section, and a generally planar flange portion 30 extending in a plane outwardly of the main, neck and head portions and sealed to said top surface 18 of the backing sheet 16, preferably by means of a vacuum seal formed by heat and pressure. The main, neck and head portions extend outwardly of top surface 18, and bound therewith a compartment 32 in which the pellets 12 are contained with clearance.

The packet 14 is provided with a frangible zone 34 which, when broken, enables removal of the pellets 12 from the compartment 12 and extends across the neck portion 26 at the juncture between the neck and head portions, i.e., where the covering member has its smallest cross-section.

As previously mentioned, the backing sheet has a certain degree of frangibility and is bendable when subjected to external forces at the neck portion. The covering member has a degree of rigidity and, hence, frangibility greater than that of the backing sheet so that the covering member fractures at the neck portion when external forces, e.g., fingertip pressure, bend the backing sheet. Indeed, in use, the synthetic plastic material of the covering member does not substantially exhibit plastic deformation, but, instead, cracks open, whereas, the paper backing sheet merely creases. The covering member actually cracks into two fractured parts, both of which remain attached to the bent backing sheet due to the presence of the flange portion 30. Once cracked open, the frusto-conically-shaped neck portion has tapered walls bounding a pouring spout through which the pellets may pass for administration of the pharmaceutical preparation. The light-transmissive covering member permits the patient to view the pellets through the covering member to evaluate how many of the pellets have been removed from the compartment.

The juncture between the neck 26 and the head 28 portions of the covering member is sufficient, due to its reduced cross-section, to constitute the frangible zone at which the covering member is fractured. In some cases, it may be desirable to facilitate the breaking operation by further weakening the frangible zone by providing a crease line across the rear of the backing sheet 16 immediately behind the aforementioned juncture. As shown in FIG. 4, the crease line can also be constituted by a series of linear perforations 36, none of which goes entirely through the backing sheet in order to maintain the integrity and seal of the compartment 32.

Also shown in FIG. 4 is a set of exemplary indicia applied, e.g., by printing, onto the bottom surface 20 of the backing sheet 16. The indicia may include the identification of the drug, instructions as to how to break open the packet, the dosage amount of the pharmaceutical preparation within the packet, directions for use, the expiration date, a warning notice, and any other information which the drug manufacturer wishes to impart to the patient. The indicia need not be applied only to the bottom surface 20 of the backing sheet; they could equally as well be applied to the front surface thereof, or to the covering member.

As shown in FIG. 5, more than one breakable packet can be attached to the same backing sheet. Thus, packets 14, 14A are both provided on a backing sheet 38 in an analogous manner to that described earlier for backing sheet 16, except that the backing sheet 38 is provided with a V-shaped notch 40 to permit ready detachment of each packet from the backing sheet 38 by tearing along the notch 40. Alternatively, each of the packets 14, 14A may be separately opened for removal of the pellets by rupturing the juncture between the neck 26 and head 28 on each packet when desired, without separation of the backing sheets of the joined packets.

This invention also contemplates the provision of more than two packets on a single backing sheet 38 and, indeed, the packets can be mounted on a continuous strip wound in a roll, or on a large sheet, each packet being detachable from the strip by any convenient tear-off means, or being separately rupturable for removal of the pelletized medication.

Turning, then, to FIG. 3, each pellet contains a core 42 of a pharmaceutical preparation comprising one or more active ingredients, and pharmaceutically acceptable excipients, binders and fillers, if any, as well as a non-tacky outer coating 44. Each peller has an average total diameter of not greater than 1 mm. Each non-tacky coating includes a polymeric material, which advantageously comprises a polymer which is a cationic copolymeric acrylic resin based on methacrylate and neutral methacrylic acid esters, and a basic compound filler. An example of such cationic copolymeric acrylic resin is "EUDRAGIT E" (Pharma International).

As a basic compound, any organic or inorganic compound having a high degree of solubility in an acidic medium may be used and, as preferred embodiments, calcium carbonate, aluminum hydroxide or magnesium carbonate may be mentioned. The filler is preferably present in an amount from about 10% to about 90% by weight of the coating, preferably in a range from about 30% to about 60% by weight and, still more preferably, in amount about 50% by weight. The filler reduces the amount of polymer that is used, greatly reducing manufacturing costs. The filler reduces the tackiness of the polymer to not only reduce manufacturing difficulties in coating, but also promotes the dissolution of the pellets in the gastric juices of a patient's stomach, as heretofore described.

A major purpose of the coating is also to mask the taste of the pharmaceutical preparation in the cores of the pellets and, accordingly, to delay the dissolution of the pellets until they have reached the patient's stomach.

Another embodiment of this invention involves utilizing as the polymeric material in the pellet coating a water-soluble polymer such as gelatin or hydrogel compounds. Examples of such water-soluble polymers are acrylamide, N-vinyl pyrolidone and N,N'-methylenebisacrylamide.

The pharmaceutical preparation itself can comprise any orally-active, gut-absorbably active ingredient including, by way of example, analgesics, such as acetaminophen, aspirin, ibuprofen, morphine; antiasthmatics,such as theophylline, albuterol, prednisone, prednisolone; antimicrobials (antibacterials, antibiotics, antifungal agents), such as sulfa drugs, trimethoprim, nitrofurantoin, penicillins, cephalosporins, tetracyclines, chloramphenicol, erythromycin, griscofulvin, nystatin; antihistamines, such as phenylpropranolamine, pseudoephedrine, clemastine, terfenadine; anti-inflammatory agents, such as phenylbutazones, salicylates, steroids, naproxen, piroxicam, indomethacin, ketoprofen, sulindac; antiepileptic agents, such as valproic acid, carbamazepine; cough and cold medicines, such as dextromethorphen, guaifenesin, chlorphenitamine, ammonium chloride; cardiovascular agents, such as labetolol, propranolol, timolol, verapamil, diltiazem, nifedipine, procainamide, guinidines; diuretics, such as furosemide, thiazide, spironolactone; laxatives, such as docusate, bisacodyl; tranquilizers, such as lorazepam, prazepam, diazepam, chlordiazepoxide, hydroxyzine, meprobamate, phenothiazines; vitamins.

The backing sheet need not be constituted solely of paper, but may be coated with a plastic or aluminum layer.

In practice, the novel pharmaceutical dispensing arrangement is provided to a health professional or to the patient who administers the pharmaceutical preparation by rupturing one or more of the individual dosage packets and pouring the contents into the mouth of the patient for immediate swallowing, with or without water or other accompanying liquid. The pellets would normally be self-administered into the mouth, except in the case of small children, elderly or incapacitated patients, or drug addicts, psychiatric patients and others who may conceal or not take their medication if self-administration is permitted.

## Claims

1. A package [10] for administering a pharmaceutical preparation, including a breakable packet [14] having a backing sheet [16] and a covering member [22] overlying the backing sheet [16] and bounding therewith a compartment [32] in which the pharmaceutical preparation is contained, the covering member [22] including a hollow main portion [24] extending along a longitudinal direction, a neck portion [26] adjacent the main portion [24], a head portion [28] adjacent the neck portion [26], and a frangible zone [34] which, when broken, opens the compartment [32] for discharging of the pharmaceutical preparation,

characterised in that:

[a] the pharmaceutical preparation is contained in a plurality of pellets [12] having non-tacky coatings [44];

[b] the neck portion [26] has tapered walls which converge toward each other along the longitudinal direction to a waist;

[c] the head portion [28] has tapered walls which diverge away from each other along the longitudinal direction away from the waist;

[d] the covering member [22] has its smallest dimension, as considered along a transverse direction generally perpendicular to the longitudinal direction, at the waist across which the frangible zone [34] extends; and

[e] the neck portion [26] forms a tapered pouring spout through which the pellets [12] are poured from the opened compartment [32].

2. The package [10] as recited in Claim 1, wherein the covering member [22] has a predetermined degree of frangibility, and wherein the back sheet [16] has a degree of frangibility less than said predetermined degree of frangibility.

3. The package [10] as recited in Claim 2, wherein the backing sheet [16] is constituted of a paper material coated with plastic or aluminium, and wherein the covering member [12] is constituted of a synthetic plastic material.

4. The package [10] as recited in Claim 1, wherein the backing sheet [16] has opposite major planar surfaces [18, 20], and wherein the covering member [22] extends outwardly of, and is sealed to, one [18] of said major surfaces.

5. The package [10] as recited in Claim 4, wherein the hollow main portion [24] has a predetermined half-cylindrical cross-section, wherein the neck portion [26] has a half-frusto-conical cross-section less than said predetermined cross-section, wherein the head portion [28] has a half-spherical cross-section, and wherein the covering member [22] has a flange portion [30] sealed to said one major surface [18].

6. The package [10] as recited in Claim 5, wherein the backing sheet [16] is bendable when subjected to external forces at the waist, and wherein the covering member [22] fractures at the waist when said external forces bend the backing sheet [16].

7. The package [10] as recited in Claim 6, wherein the covering member [22] fractures into fractured parts which remain attached to the bent backing sheet [16], one fractured part being the head portion [28], and another fractured part being the main [14] and neck [26] portions.

8. The package [10] as recited in Claim 4, wherein the covering member [22] is constituted of a light-transmissive material to permit viewing of the pellets [12] through the covering member [22].

9. The package [10] as recited in Claim 4, wherein the packet [14] includes indicia applied onto the other [20] of said major surfaces of the backing sheet [16].

10. The package [10] as recited in Claim 1; and further comprising another covering member [22] overlying the backing sheet [16] and bonding therewith another compartment [32] in which additional pellets [12] are contained.

11. The package [10] as recited in Claim 1, wherein each pellet [12] has a diameter not greater than 1mm, said pellets [12] being freely accommodated with clearance in the compartment [32].

12. The package [10] as recited in Claim 1, wherein each non-tacky coating [44] includes a polymeric material.

13. The package [10] as recited in Claim 12, wherein each non-tacky coating [44] further includes a basic compound filler that is soluble in acid.

14. The package [10] as recited in Claim 12, wherein the polymeric material includes a cationic co-polymeric acrylate resin based on methacrylate and neutral methacrylic acid esters.

15. The package [10] as recited in Claim 13, wherein the basic compound filler is selected from the group consisting of calcium carbonate, aluminium hydroxide and magnesium carbonate.

16. The package [10] as recited in Claim 13, wherein the basic compound filler is present in the coating [44] in an amount from about 10% to about 90% by weight

17. The package [10] as recited in Claim 12, wherein the polymeric material is selected from the group consisting of cellulosic, polyacrylate and poly-vinyl-alcoholic polymers.

18. The package [10] as recited in Claim 1, wherein the pharmaceutical preparation contained in the pellets [12] is a fraction of a dose to be administered to a human patient.

19. The package [10] as recited in Claim 1, wherein the pharmaceutical preparation contained in the pellets [12] is a multiple of a dose to be administered to a human patient.

20. The package [10] as recited in Claim 1, wherein the pharmaceutical preparation comprises an active ingredient and pharmaceutically acceptable binders, fillers or excipients.

21. The package [10] as recited in Claim 20, wherein said active ingredient is orally active.

22. The package [10] as recited in Claim 21, wherein said orally active ingredient is selected from the group consisting of analgesics, antiasthmatics, antimicrobials, antibacterials, antibiotics, antifungal agents, antihistamines,, anti-inflammatory agents, antiepileptic agents, cough and cold medicines, cardiovascular agents, diuretics, laxatives, tranquilliser, and vitamins.

## Patentansprüche

1. Packung (10) zum Verabreichen einer Arzneimittelzubereitung, mit einem aufbrechbaren Päckchen (14) mit einer dünnen Rückplatte (16) und einem Deckteil (22), welches über der dünnen Rückplatte (16) liegt und mit dieser einen Zwischenraum (32) begrenzt, in dem die Arzneimittelzubereitung enthalten ist, wobei das Deckteil (22) einen hohlen Hauptabschnitt (24), der sich entlang einer Längsrichtung erstreckt, einen Halsabschnitt (26), benachbart dem Hauptabschnitt (24), einem Kopfabschnitt (28), benachbart dem Halsabschnitt (26), und einem Bruchbereich (31), welcher, wenn er aufgebrochen wird, den Zwischenraum (42) zum Ausgeben der Arzneimittelzubereitung öffnet, umfaßt, dadurch **gekennzeichnet**, daß:

a) die Arzneimittelzubereitung in einer Vielzahl von Pillen (12) mit klebfreien Überzügen (44) enthalten ist;

b) der Halsabschnitt (26) abgeschrägte Wände hat, die aufeinander zu in Längrichtung zu einem Bauch zusammenlaufen;

c) der Kopfabschnitt (28) abgeschrägte Wände hat, die voneinander in Längsrichtung weg von dem Bauch laufen;

d) das Deckteil (22) seine kleinsten Abmessungen, betrachtet längs einer Querrichtung im wesentlichen senkrecht zur Längsrichtung, an dem Bauch hat, über den sich der Aufteißbereich (34) erstreckt; und

e) der Halsabschnitt (26) eine abgeschrägte Ausgießtülle bildet, durch die die Pillen (12) aus dem geöffneten Zwischenraum (26) gegossen werden.

2. Packung (10) nach Anspruch 1, bei der das Deckteil (22) einen vorbestimmten Grad von Bruchfähigkeit hat und bei dem die dünne Rückplatte (16) einen Grad an Bruchfähigkeit hat, der geringer ist als der vorbestimmte Grad an Bruchfähigkeit.

3. Packung (10) nach Anspruch 2, bei der die dünne Rückplatte (16) aus einem Papiermaterial, das mit Kunststoff oder Aluminium überzogen ist, gebildet ist, und bei dem das Deckteil (12) aus einem synthetischen Kunststoffmaterial gebildet ist.

4. Packung (10) nach Anspruch 1, bei dem die dünne Rückplatte (16) einander gegenüberliegende ebene Hauptflächen (18, 20) aufweist und bei dem das Deckteil (22) sich von einer (18) der Hauptflächen nach außen erstreckt und mit dieser versiegelt ist.

5. Packung (10) nach Anspruch 4, bei dem der hohle Hauptabschnitt (24) einen vorbestimmten halbzylindrischen Querschnitt hat, bei dem der Halsabschnitt (26) einen halb-kegelstumpfförmigen Querschnitt hat, geringer als der vorbestimmte Querschnitt, bei dem der Kopfabschnitt (28) einen halbkugelförmigen Querschnitt hat und bei dem das Deckteil (22) einen Anschlußbereich (30) hat, der mit einer der Hauptflächen (18) versiegelt ist.

6. Packung (10) nach Anspruch 5, bei dem die dünne Rückplatte (16) biegbar ist, wenn sie am Bauch äußeren Kräften ausgesetzt ist, und bei dem das Deckteil (22) am Bauch bricht, wenn die äußeren Kräfte die dünne Rückplatte (16) biegen.

7. Packung (10) nach Anspruch 6, bei dem das Deckteil (22) in Bruchteile bricht, die an der gebogenen dünnen Rückplatte (16) befestigt bleiben, wobei eine Bruchteil der Kopfabschnitt (28) und ein anderer Bruchteil der Haupt-(14) und der Hals-(26)Abschnitt ist.

8. Packung (10) nach Anspruch 4, bei dem das Deckteil (22) aus lichtdurchlässigem Material gebildet ist, um zu erlauben, daß man die Pillen (12) durch das Deckteil (22) sieht.

9. Packung (10) nach Anspruch 4, bei dem das päckchen (14) Kennzeichnungen umfaßt, die auf die andere (20) der Hauptflächen der dünnen Rückplatte (16) aufgebracht sind.

10. Packung (10) nach Anspruch 1, welche weiterhin ein weiteres Deckteil (20) aufweist, das übet der dünnen Rückplatte (16) liegt und damit einen anderen Zwischenraum (32) begrenzt, in welchem zusätzliche Pillen (12) enthalten sind.

11. Packung (10) nach Anspruch 1, bei dem jede Pille (12) einen Durchmesser hat, der nicht größer als 1 mm ist, wobei die Pillen (12) in dem Zwischenraum (32) frei mit Abstand untergebracht sind.

12. Packung (10) nach Anspruch 1, bei dem jeder klebfreie Überzug (44) ein polymeres Material umfaßt.

13. Packung (10) nach Anspruch 12, bei dem jeder klebfreie Überzug (44) weiterhin einen basischen Verbundfüller umfaßt, der in Säure löslich ist.

14. Packung (10) nach Anspruch 12, bei dem das polymere Material ein kationisches copolymeres Acrylat auf der Grundlage von Methacrylat und neutralen Methacrylsäuye-Estern umfaßt.

15. Packung (10) nach Anspruch 13, bei dem der basische Verbundfüller aus der Gruppe aus Kalziumkarbonat, Aluminiumhydroxid und Magnesiumkarbonat ausgewählt ist.

16. Packung (10) nach Anspruch 13, bei dem der basische Verbundfüller in dem Überzug (44) in einer Menge von etwa 10 Gew-% bis etwa 90 Gew-% vorliegt.

17. packung (10) nach Anspruch 12, bei dem das polymere Material aus der Gruppe aus Zellulose, polyacrylat und polyvinylalkohol-polymeren ausgewählt ist.

18. Packung (10) nach Anspruch 1, bei dem die Arzneimittelzusammensetzung, die in den Pillen (12) enthalten ist, ein Bruchteil einer Dosis ist, die einem menschlichen Patienten verabreicht werden soll.

19. Packung (10) nach Anspruch 1, bei dem die Arzneimittelzusammensetzung, die in den Pillen (12) enthalten ist, ein Vielfaches einer Dosis ist, die einem menschlichen Patienten verabreicht werden soll.

20. Packung (10) nach Anspruch 1, bei dem die Arzneimittelzusammensetzung einen aktiven Bestandteil und pharmazeutisch akzeptable Binder, Füller oder Vehikel umfaßt.

21. Packung (10) nach Anspruch 20, bei dem der aktive Bestandteil oral aktiv ist.

22. Packung (10) nach Anspruch 21, bei dem der oral aktive Bestandteil aus der Gruppe aus Analgetika, Antiasthmatika, antimikrobischen Mitteln, antibakteriellen Mitteln, Antibiotika, antifungalen Mitteln, Antihistaminen, Mitteln gegen Entzündungen, antiepileptischen Mitteln, Husten- und Erkältungsarzneien, cardiovaskularen Mitteln, Diuretika, Abführmitteln, Beruhigungsmitteln und Vitaminen ausgewählt ist.

## Revendications

1. Conditionnement [10] en vue de l'administration d'une préparation pharmaceutique, comprenant un paquet cassable [14] qui présente une feuille de support [16] et un élément de couverture [22] recouvrant la feuille de support [16] et délimitant avec elle un compartiment [32] dans lequel est contenue la préparation pharmaceutique, l'élément de couverture [22] comprenant une partie principale creuse [24] s'étendant le long d'une direction longitudinale, une partie de col [26] adjacente à la partie principale [24], une partie de tête [28] adjacente à la partie de col [26], et une zone de moindre résistance [34] laquelle, une fois rompue, ouvre le compartiment [32] en vue de la libération de la préparation pharmaceutique, caractérisé par le fait que :

[a] la préparation pharmaceutique est contenue dans une pluralité de pastilles [12] ayant des revêtements non-collants [44] ;

[b] la partie de col [26] a des parois coniques qui convergent les unes vers les autres le long de la direction longitudinale jusqu'à une partie d'étranglement ;

[c] la partie de tête [28] a des parois coniques qui divergent les unes des autres le long de la direction longitudinale à l'opposé de la partie d'étranglement ;

[d] l'élément de couverture [22] a sa dimension la plus petite, telle que considérée le long d'une direction transversale généralement perpendiculaire à la direction longitudinale, au niveau de la partie d'étranglement à travers laquelle s'étend le zone de moindre résistance [34] ; et

[e] la partie de col [26] forme un bec-verseur conique à travers lequel les pastilles [12] sont versées à partir du compartiment ouvert [32].

2. Conditionnement [10] selon la revendication 1, dans lequel l'élément de couverture [22] a un degré prédéterminé de moindre résistance, et dans lequel la feuille de support [16] a un degré de moindre résistance inférieur audit degré prédéterminé de moindre résistance.

3. Conditionnement [10] selon la revendication 2, dans lequel la feuille de support [16] est constituée d'une matière à base de papier, revêtue de matière plastique ou d'aluminium, et dans lequel l'élément de couverture [22] est constitué d'une matière plastique synthétique.

4. Conditionnement [10] selon la revendication 1, dans lequel la feuille de support [16] a des surfaces planes majeures opposées [18,20], et dans lequel l'élément de couverture [22] s'étend extérieurement à, et est scellé à, l'une [18] desdites surfaces majeures.

5. Conditionnement [10] selon la revendication 4, dans lequel la partie principale creuse [24] a une section transversale semi-cylindrique, dans lequel la partie de col [26] a une section transversale semi-tronconique inférieure à ladite section transversale prédéterminée, dans lequel la partie de tête [28] a une section transversale hémisphérique, et dans lequel l'élément de couverture [22] a une partie formant bride [30] scellée à ladite surface majeure précitée [18].

6. Conditionnement [10] selon la revendication 5, dans lequel la feuille de support [16] est pliable lorsqu'elle est soumise à des forces externes au niveau de la partie d'étranglement, et dans lequel l'élément de couverture [22] se casse au niveau de la partie d'étranglement lorsque lesdites forces externes plient la feuille de support [16].

7. Conditionnement [10] selon la revendication 6, dans lequel l'élément de couverture [22] se casse en morceaux brisés qui restent attachés à la feuille de support pliée [16], l'un des morceaux brisés étant la partie de tête [28], et l'autre morceau brisé étant les parties principale [24] et de col [26].

8. Conditionnement [10] selon la revendication 4, dans lequel l'élément de couverture [22] est constitué d'un matériau translucide pour permettre de voir les pastilles [12] à travers l'élément de couverture [22].

9. Conditionnement [10] selon la revendication 4, dans lequel le paquet [14] comprend un timbre imprimé appliqué sur l'autre [20] desdites surfaces majeures de la feuille de support [16].

10. Conditionnement [10] selon la revendication 1 ; et comprenant en outre un autre élément de couverture [22] qui recouvre la feuille de support [16] et auquel est fixé un autre compartiment [32] dans lequel sont contenues des pastilles additionnelles [12].

11. Conditionnement [10] selon la revendication 1, dans lequel chaque pastille [12] a un diamètre non supérieur à 1 mm, lesdites pastilles [12] étant disposées librement avec jeu dans le compartiment (32), sans l'occuper totalement.

12. Conditionnement [10] selon la revendication 1, dans lequel chaque revêtement non-collant [44] comprend une matière polymère.

13. Conditionnement [10] selon la revendicaion 12, dans lequel chaque revêtement non-collant [44] comprend en outre une charge de type composé basique qui est soluble dans les acides.

14. Conditionnement [10] selon la revendication 12, dans lequel la matière polymère comprend une résine d'acrylate copolymère cationique à base de méthacrylate et d'esters d'acide méthacrylique neutres.

15. Conditionnement [10] selon la revendication 13, dans lequel la charge de type composé basique est choisie dans le groupe constitué par le carbonate de calcium, l'hydroxyde d'aluminium et le carbonate de magnésium.

16. Conditionnement [10] selon la revendication 13, dans lequel la charge de type composé basique est présente dans le revêtement [44] dans une quantité d'environ 10% à environ 90% en poids.

17. Conditionnement [10] selon la revendication 12, dans lequel la matière polymère est choisie dans le groupe constitué par les polymères cellulosiques, les polymères de type polyacrylates et les polymères de type alcools polyvinyliques.

18. Conditionnement [10] selon la revendication 1, dans lequel la préparation pharmaceutique contenue dans les pastilles [12] est une fraction d'une dose devant être administrée à un patient humain.

19. Conditionnement [10] selon la revendication 1, dans lequel la préparation pharmaceutique contenue dans les pastilles [12] est un multiple d'une dose devant être administrée à un patient humain.

20. Conditionnement [10] selon la revendication 1, dans lequel la préparation pharmaceutique comprend un ingrédient actif et des liants, charges ou excipients pharmaceutiquement acceptables.

21. Conditionnement [10] selon la revendication 20, dans lequel ledit ingrédient actif est actif par voie orale.

22. Conditionnement [10] selon la revendication 21, dans lequel ledit ingrédient actif par voie orale est choisi dans le groupe constitué par les analgésiques, les anti-asthmatiques, les substances anti-microbiennes, les bactéricides, les antibiotiques, les agents antifongiques, les anti-histaminiques, les agents anti-inflammatoires, les agents anti-épileptiques, les médicaments contre la toux et le rhume, les agents cardio-vasculaires, les diurétiques, les laxatifs, les tranquillisants et les vitamines.

**FIG. 1**

**FIG. 2**

**FIG. 5**

**FIG. 3**

**FIG. 4**

DRUG
NAME

TEAR
HERE

DOSAGE
USE
DIRECTIONS
WARNINGS

EXPIRES
MONTH/YEAR